Europäisches Patentamt

European Patent Office

Office européen des brevets

(19)

(11) Numéro de publication : **0 294 257 B1**

(12) **FASCICULE DE BREVET EUROPEEN**

(45) Date de publication du fascicule du brevet :
09.10.91 Bulletin 91/41

(51) Int. Cl.⁵ : **A61F 5/441**

(21) Numéro de dépôt : **88401129.7**

(22) Date de dépôt : **09.05.88**

(54) Filtre à évent de dégazage incorporé, destiné à être monté dans des poches de drainage d'anus artificiels au cours de leur fabrication.

(30) Priorité : **14.05.87 FR 8706797**

(43) Date de publication de la demande :
**07.12.88 Bulletin 88/49**

(45) Mention de la délivrance du brevet :
**09.10.91 Bulletin 91/41**

(84) Etats contractants désignés :
**DE ES GB IT**

(56) Documents cités :
**EP-A- 0 037 944**
**EP-A- 0 064 044**
**EP-A- 0 068 964**
**EP-A- 0 235 928**
**FR-A- 2 350 832**
**US-A- 4 367 742**

(73) Titulaire : **LABORATOIRES BIOTROL S.A.**
**1, rue du Foin**
**F-75140 Paris Cedex 03 (FR)**

(72) Inventeur : **Holtermann, Henri**
**12 Avenue Pierre Loti**
**F-64500 St-Jean de Luz (FR)**

(74) Mandataire : **Phélip, Bruno et al**
**c/o Cabinet Harlé & Phélip 21, rue de La**
**Rochefoucauld**
**F-75009 Paris (FR)**

Jouve, 18, rue Saint-Denis, 75001 PARIS

## Description

La présente invention concerne un nouveau filtre adsorbant pour poches de drainage d'anus artificiels avec évent de dégazage sur le filtre.

Les patients ayant subi une opération chirurgicale, par exemple une colostomie ou une iléostomie avec création d'anus artificiel sont généralement équipés de poches de recueil réalisées en un matériau souple, par exemple une matière plastique, lesdites poches étant composées de feuilles ou films de ce matériau, imperméables aux gaz et aux odeurs, soudées entre elles sur leur périphérie et dont l'une comporte une ouverture frontale appropriée pour assurer une communication et une liaison pratiquement étanche entre l'anus artificiel et l'intérieur de la poche.

Les excrétions solides ou liquides émises et recueillies dans cette poche s'accompagnent de l'émission de gaz intestinaux dont l'évacuation est rendue nécessaire de façon à éviter un gonflage excessif de ladite poche. Il convient toutefois, dans l'intérêt du confort du patient et surtout pour éviter que les deux films constitutifs de la poche ne se collent l'un à l'autre, de façon à former un étranglement préjudiciable à une utilisation convenable de la poche, de maintenir à l'intérieur de celle-ci un minimum de pression résiduelle. Par ailleurs, étant donné que certains gaz intestinaux dégagent une odeur nauséabonde gênante pour le patient et son entourage, il est avantageux de munir les poches de drainage d'anus artificiels d'un filtre adsorbant placé sur le trajet des gaz de telle sorte que ceux-ci le traversent pour se purifier avant de sortir de la poche.

Les filtres actuellement utilisés sont soit fixés extérieurement ou intérieurement sur l'un des deux films constitutifs de la poche avant la réalisation de celle-ci soit rapportés sur la face externe de l'un des deux films constitutifs de la poche. Dans l'un comme dans l'autre de ces deux cas, des variantes ont été réalisées dans le but d'obtenir des filtres plus efficaces, tout en leur conservant un volume limité. Pour certaines de ces variantes, il s'agit de faire dévier radialement les gaz à l'intérieur de la pastille du filtre, dans laquelle ils ont pénétré soit par la périphérie, soit par un orifice central, ou bien encore de faire suivre aux gaz un parcours complexe par un jeu de déviations et de chicanes à l'intérieur de la pastille du filtre, qui présente la forme d'un disque ou d'un anneau.

Dans la pratique, le filtre doit toujours rester de faible épaisseur, de manière à avoir l'incidence la plus faible possible sur l'épaisseur hors-tout de la poche. Il doit cependant avoir un pouvoir filtrant et une capacité d'adsorption des odeurs suffisante pour assurer au porteur d'une poche munie d'un tel filtre une autonomie allant d'un minimum de quelques heures à une durée aussi longue que 12 heures, 24 heures et même plus si possible. S'il s'agit d'un filtre incorporé à la poche lors de sa fabrication, il doit en tout état de cause conserver toute son efficacité aussi longtemps que l'appareillage demeure en place. Pourtant les utilisateurs réclament de plus en plus des poches avec filtres pré-incorporés. Il s'agit surtout de patients d'un certain âge, qui n'ont pas l'habilité requise pour effectuer eux-mêmes la pose et la mise en service, souvent délicates, de filtres séparés. Quel que soit le type des filtres pour poche de drainage actuellement sur le marché, ceux-ci sont en règle générale d'une autonomie limitée à 12 heures au maximum, le plus souvent à 6 heures et même à quelques heures seulement en ce qui concerne les émissions de gaz particulièrement malodorantes. On peut considérer ces durées de fonctionnement comme insuffisantes, notamment quand il s'agit d'appareillages vidangeables, puisque le patient est alors contraint de changer un appareillage dont la partie collectrice est pourtant encore utilisable.

Le brevet français 8111757 du 15 Juin 1981 au nom de la même demanderesse, qui correspond à la demande de brevet européen EP-A-0068964 concerne un système de filtre et d'évent intégré dans des poches de drainage d'anus artificiels au cours de la fabrication de celles-ci, en association avec une partie de la zone périphérique de soudure des films constitutifs de la poche, en réalisant ainsi un filtre à passage direct dont la face de sortie débouche à l'extérieur de la poche et/ou à l'intérieur d'une chambre aval qui fait partie intégrante de la poche ou la complète et dont les parois peuvent être perforées pour réaliser un évent.

Dans son certificat d'addition au brevet ci-dessus, la demanderesse a décrit et revendiqué un système perfectionné de filtres et d'évent pour poches de drainage, notamment pour colostomisés et iléostomisés, qui constitue un perfectionnement au système décrit dans le brevet précité et qui, tout en restant extrêmement simple, peut être incorporé à de telles poches, procure le moyen d'ajuster la durée de vie efficace du système de façon qu'elle corresponde au moins à la durée d'utilisation prévue de la poche, et permet en outre à l'utilisateur de déterminer lui-même à convenance le débit de sortie de gaz à donner à ce système à défaut d'une pré-perforation pratiquée à la fabrication.

On connaît encore, par ailleurs, par les documents US-4367.742 et EP-64044 représentant l'état de la technique le plus proche de l'invention, des filtres à évent de dégazage incorporé, destinés à être montés dans des poches de drainage d'anus artificiels au cours de la fabrication de celles-ci, en association avec une partie de la zone périphérique de soudure des films constitutifs de la poche et constituant un filtre à passage direct. Ces filtres sont chacun constitués d'un corps sensiblement oblong méplat comportant un support central ou âme constitué d'un tissu non tissé ou d'une matière plastique expansée à alvéoles ouvertes, imprégné d'une matière active adsorbante et recouvert d'une partie des films constitutifs de la poche en matière plastique imperméable au gaz, ce filtre comportant au moins un évent de dégazage sur une de ces faces, pour mettre en communication ledit filtre et l'intérieur de la poche. Le filtre dont fait état le document US-4367742 est en outre également muni d'un raccord tubulaire d'évacuation dont une extrémité est située au niveau du filtre et qui débouche à l'air libre par son autre extrémité. Le filtre du document EP 64044 est quant à lui disposé au niveau d'au moins un coin de la pochette, lequel soin est destiné à être déchiré pour constituer un évent d'évacuation à l'air libre.

Un objet de la présente invention est de réaliser un système de filtre à évent de dégazage incorporé, qui tout en conservant les qualités inhérentes aux systèmes de filtre déjà mis au point par la demanderesse et analysés ci-dessus, présente une conception particulière lui permettant une meilleure application dans les poches de drainage d'anus artificiels dans lesquelles il est monté en cours de fabrication.

La présente invention a donc pour objet un filtre à évent de dégazage incorporé ; destiné à être monté dans des poches de drainage d'anus artificiels au cours de la fabrication de celles-ci, en association avec une partie de la zone périphérique de soudure des films constitutifs de la poche, ce filtre constituant un filtre à passage direct et étant constitué d'un corps oblong méplat comportant un support central ou âme constitué d'un tissu non tissé ou d'une matière plastique expansée à alvéoles ouvertes, et imprégné d'une matière active adsorbante et recouverte d'au moins un film protecteur, ce filtre comportant au moins un évent de dégazage sous la forme d'une fenêtre prévue sur une des faces longitudinales du filtre et étant caractérisé en ce que le film protecteur laisse libres les extrémités terminales dudit filtre destinées à être incorporées à la zone périphérique de la poche de drainage.

En fait, le support de la matière active adsorbante peut être en n'importe quelle matière présentant les qualités de résistance mécanique et aux agents chimiques acceptables, par exemple un polyuréthane expansé, un polyester expansé ou un polyéther expansé. La matière adsorbante peut être constituée par n'importe quel composé présentant les caractéristiques d'adsorption convenant à l'usage, par exemple un métal ou un métalloïde, ou un oxyde métallique ou un complexe organométallique ou bien un mélange métal et/ou métalloïde et/ou oxyde métallique et/ou complexe organométallique. On peut envisager avantageusement d'utiliser du charbon actif ou du ricinoléate de zinc.

L'invention s'étend également à toutes les poches de drainage d'anus artificiels incorporant le système de filtre décrit ici et dans ce qui suit.

D'autres avantages et caractéristiques de la présente invention apparaîtront à la lecture de la description non limitative suivante de formes de réalisation de système de filtre à évent de dégazage incorporé, en référence aux dessins annexés dans lesquels :

Fig. 1 est une vue partielle en perspective d'un système de filtre selon la présente invention, dit "filtre méplat carboné" ;

Fig. 2 est une vue en perspective partielle d'une première variante de réalisation de système de filtres selon l'invention ;

Fig. 3 est une vue en perspective partielle d'une seconde variante de réalisation de système de filtres à évent de dégazage de l'invention ;

Fig. 4 est une vue en plan d'une mini-poche de drainage équipée d'un filtre carbone méplat selon l'invention ; et

Fig.5 est une vue en plan d'une poche de drainage dite "intégrale" équipée d'un filtre de carbone méplat selon l'invention.

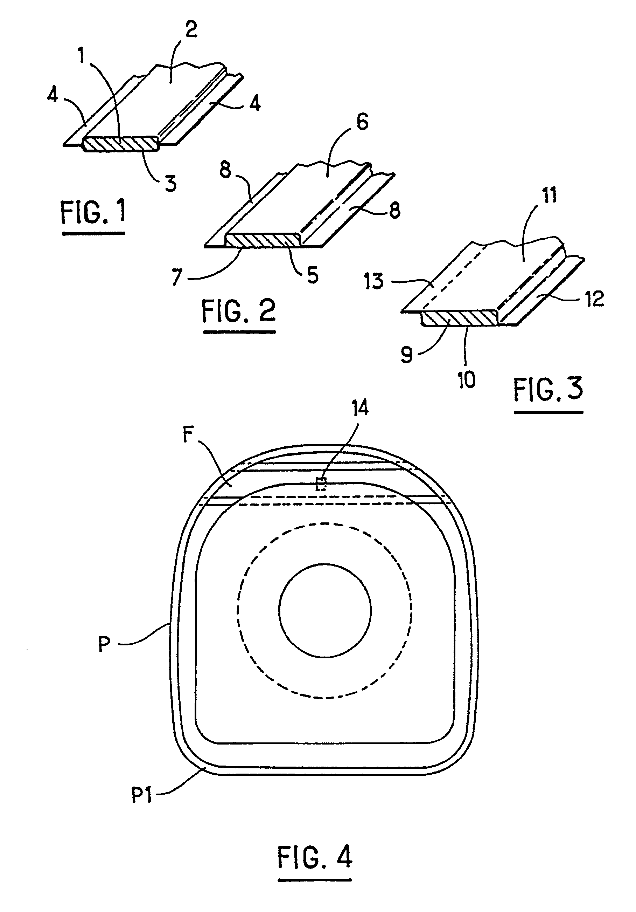

Dans la forme de réalisation de la figure 1, le filtre carbone méplat selon l'invention est constitué d'un support central ou âme 1 en matière plastique expansée à alvéoles ouvertes par exemple du polyuréthane imprégné d'une matière adsorbante, par exemple du charbon actif associé à de l'oxyde ferrique. Cette âme centrale 1 est recouverte de deux films 2, 3 en matière plastique imperméable aux gaz. Les films 2 et 3 se rejoignent sensiblement à mi-épaisseur de l'âme 1 de façon à former des zones de bord ou lisières 4.

Dans la forme de réalisation de la figure 2, le filtre méplat carbone de l'invention est constitué également d'une âme adsorbante 5 recouverte de deux films protecteurs 6 et 7 qui se rejoignent au niveau des arêtes d'une des faces longitudinales de l'âme 5 à former des lisières 8.

Dans la forme de réalisation de la figure 3, le filtre méplat carbone est aussi constitué d'une âme adsorbante interne 9 protégée par deux films imperméables 10 et 11 qui se rejoignent, d'une part, au niveau d'une arête inférieure de l'âme 9 de façon à former une lisière 12 et, d'autre part, au niveau d'une arête supérieure de ladite âme 9 de façon à former une lisière opposée 13.

Les âmes respectives 1, 5, 9 des filtres carbone méplat de l'invention sont constituées d'une mousse de polyuréthane imprégnée d'un charbon actif renfermant avantageusement 10% d'oxyde ferrique. Les films ou pellicules d'étanchéité 2, 3 ; 6, 7 ; et 10, 11 sont avantageusement constitués d'une matière plastique imperméable aux gaz.

Parmi les plastiques imperméables appropriés à l'étanchéité du filtre, on peut citer le polychlorure de vinylidène, les associations de polychlorure de vinylidène et de polyéthylène, les associations de polychlorure de vinylidène et d'éthylène acétate de vinyle, les associations de polyéthylène et de téréphtalate de polyéthylène, les associations de polyéthylène et d'alcool polyvinylique.

Sur la figure 4 est représenté un filtre F incorporé dans une poche de drainage P dite "mini poche", constituée de façon classique d'un film complexe transparent soudé périphériquement à un film complexe blanc de façon à former une zone de soudure périphérique P1. Dans la zone de pose du filtre F, les zones d'extrémité de ce dernier viennent s'insérer de façon étanche à l'intérieur de la zone périphérique P1, de façon à laisser à l'air libre les zones périphériques non protégées du filtre F. A l'intérieur de la poche P, le filtre F est pourvu d'au moins un évent de dégazage 14 ménagé sur une de ses faces.

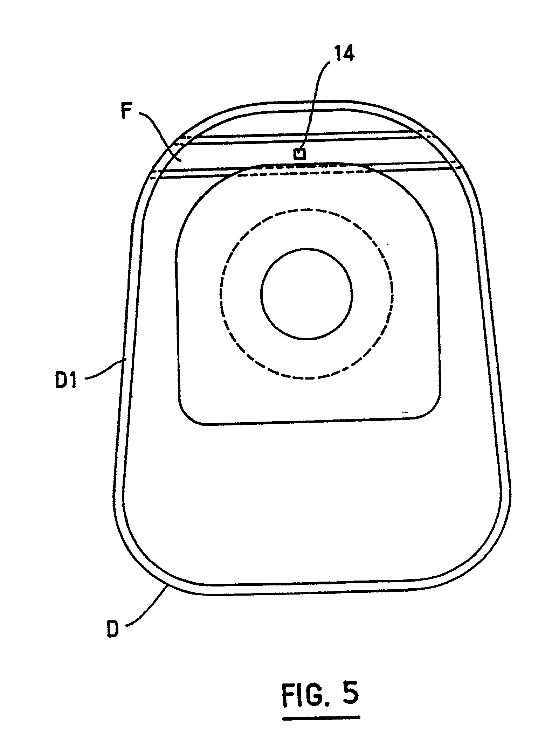

Comme représenté sur la figure 5, un filtre F présentant également un évent de dégazage 14 est également monté à l'intérieur d'une poche de drainage de plus grand volume D fabriquée de façon conventionnelle à partir d'un film transparent et d'un film blanc en plastique imperméable de façon à former une zone périphérique D1 traversée de façon étanche par les zones d'extrémité venant à l'air libre du filtre F.

Ainsi se trouve résolu selon la présente invention, le problème posé par la complexité des systèmes de filtres de dégazage, notamment au niveau de l'étanchéité et du positionnement de l'évent de dégazage sur le filtre, et ce grâce à la structure particulière du filtre carbone méplat selon la présente invention, telle que définie ci-dessus et revendiquée dans ce qui suit.

Il est clair que l'invention n'est nullement limitée aux formes de réalisation décrites ci-dessus en référence aux dessins annexés, mais qu'elle englobe toutes les modifications dans le cadre de l'invention telle que définie dans les revendications ci-dessous.

## Revendications

1. Filtre à évent de dégazage incorporé, destiné à être monté dans des poches de drainage d'anus artificiels au cours de la fabrication de celles-ci, en association avec une partie de la zone périphérique de soudure des films constitutifs de la poche et constituant un filtre à passage direct, ce filtre étant constitué d'un corps oblong méplat comportant un support central ou âme (1 ; 5 ; 9) constitué d'un tissu non tissé ou d'une matière plastique expansée à alvéoles ouvertes, imprégné d'une matière active adsorbante et recouvert d'au moins un film protecteur (2, 3 ; 6, 7, 10, 11) en matière plastique imperméable aux gaz, ce filtre comportant au moins un évent de dégazage (14) sur une de ses faces, caractérisé en ce que ledit film protecteur (2, 3 ; 6, 7 ; 10, 11) laisse libres les extrémités terminales dudit filtre destinées à être incorporées à la zone périphérique de la poche de drainage.

2. Filtre selon la revendication 1, caractérisé en ce que la matière plastique expansée à alvéoles ouvertes est du polyuréthane expansé.

3. Filtre selon l'une quelconque des revendications 1 et 2, caractérisé en ce que la matière adsorbante est un métal ou un métalloïde, ou un oxyde métallique ou un complexe organométallique ou bien un mélange métal et/ou métalloïde et/ou oxyde métallique et/ou complexe organométallique.

4. Filtre selon la revendication 3, caractérisé en ce que la matière adsorbante est du charbon actif, avantageusement imprégnée d'oxyde ferrique.

5. Filtre selon la revendication 3, caractérisé en ce que la matière adsorbante est du ricinoléate de zinc.

6. Poche de drainage pour anus artificiels, caractérisé en ce qu'elle comporte au moins un filtre selon l'une quelconque des revendications 1 à 5.

## Patentansprüche

1. Filter mit eingebauter Gasaustrittsöffnung, der dazu bestimmt ist in Ostomiebeutel für künstlichen After während deren Herstellung in Verbindung mit einem Teil der peripheren Verschweiß-Zone der die Beutel bildenden Folien montiert zu werden und einen Filter mit direkter Passage darstellt, wobei dieser Filter aus einem länglichen abgeflachten Körper besteht, der einen zentralen Träger oder Kern (1 ; 5 ; 9) enthält, welcher aus einem nichtgewebten Textilerzeugnis oder einem offenzellig aufgeschäumten Kunststoffmaterial besteht, das mit einem aktiven adsorbierenden Material imprägniert und von wenigstens einem Schutzfilm (2, 3 ; 6, 7, 10, 11) aus für Gase undurchlässigem Kunststoffmaterial überdeckt ist, wobei dieser Filter wenigstens eine Gasaustrittsöffnung (14) auf einer seiner Flächen aufweist, dadurch gekennzeichnet, daß der Schutzfilm (2, 3 ; 6, 7 ; 10, 11) die Endabschnitte des Filters freiläßt, die dazu bestimmt sind, in die Randzone des Ostomiebeutels eingebaut zu werden.

2. Filter nach Anspruch 1, dadurch gekennzeichnet, daß das offenzellig aufgeschäumte Kunststoffmaterial aufgeschäumtes Polyurethan ist.

3. Filter nach Anspruch 1 oder 2, dadurch gekennzeichnet, daß das adsorbierende Material ein Metall oder Nichtmetall oder ein Metalloxyd oder ein organometallischer Komplex oder eine Mischung aus Metallen und/oder Nichtmetallen und/oder Metalloxyden und/oder organometallischen Komplexen ist.

4. Filter nach Anspruch 3, dadurch gekennzeichnet, daß das adsorbierende Material aus Aktivkohle besteht, welches vorzugsweise mit einem Eisenoxyd imprägniert ist.

5. Filter nach Anspruch 3, dadurch gekennzeichnet, daß das adsorbierende Material aus Zink-Rizinoleat besteht.

6. Ostomiebeutel für künstlichen After, dadurch gekennzeichnet daß er wenigstens einen Filter nach einem der Ansprüche 1 bis 5 enthält.

## Claims

1. Filter with an incorporated gas escape opening, intended to be fitted in artificial anus drainage bags during the manufacture of the latter, in connection with a part of the peripheral zone of welding of the component films of the bag and constituting a direct-passage filter, this filter being made up of a flattened oblong body comprising a central support or core sheet (1 ; 5 ; 9) made up of a nonwoven fabric or of an open-cell expanded plastic material, impregnated with an adsorbent active material and covered by at least one protective film (2, 3 ; 6, 7, 10, 11) of plastic material impermeable to gases, this filter comprising at least one gas escape opening (14) on one of its faces characterised in that said protective film (2, 3 ; 6, 7 ; 10, 11) leaves free the end edges of said filter intended to be incorporated in the peripheral zone of the drainage bag.

2. Filter according to Claim 1, characterised in that the open-cell expanded plastic material is expanded polyurethane.

3. Filter according to any one of Claims 1 and 2, characterised in that the adsorbent material is a metal or a metalloid, or a metallic oxide or an organometallic complex or else a mixture of metal and/or metalloid and/or metallic oxide and/or organometallic complex.

4. Filter according to Claim 3, characterised in that the adsorbent material is activated carbon, advantageously impregnated with ferric oxide.

5. Filter according to Claim 3, characterised in that the adsorbent material is zinc ricinoleate.

6. Drainage bag for artificial anuses, characterised in that it comprises at least one filter according to any one of Claims 1 to 5.

FIG. 1

FIG. 2

FIG. 3

FIG. 4

FIG. 5